# EUROPÄISCHE PATENTANMELDUNG

(11) **EP 4 527 419 A1**
(43) Veröffentlichungstag der Anmeldung: **26.03.2025**
(21) Anmeldenummer: 23198163.0
(22) Anmeldetag: 19.09.2023
(51) Int. Cl.: A61L 2/26, G01K 13/00

(54) **ANLAGE FÜR DIE STERILISATION UND REINIGUNG VON MEDIZINISCHEN UND PHARMAZEUTISCHEN GÜTERN SOWIE VERFAHREN ZUM STEUERN EINER ANLAGE**

(71) Anmelder: Belimed Life Science AG, 8583 Sulgen (CH)
(72) Erfinder: Stark, Adrian, 9204 Andwil (CH)
(74) Vertreter: Hepp Wenger Ryffel AG

(57) **Zusammenfassung**

Die Erfindung betrifft eine Anlage (18) für die Sterilisation und/oder Reinigung von medizinischen und pharmazeutischen Gütern. Die Anlage (18) umfasst eine Kammer (21). Die Kammer (21) umfasst eine Medieneinbringung (22) sowie eine Aktorregelung (23). Mit der Aktorregelung (23) ist eine physikalische Grösse in der Kammer (21) regelbar. In der Kammer (21) ist ein Sensor (11, 12) angeordnet. Die Anlage (18) weist eine digitale Datenübertragungseinheit (8, 9) auf. Durch die digitale Datenübertragungseinheit (8, 9) sind Daten des Sensors (11, 12) digital zu einer Datenverarbeitungseinheit (6, 7) übertragbar. Die Daten können von der digitalen Datenübertragungseinheit (8, 9) drahtlos oder kabelgebunden übertragbar sein. Die Daten können zwischen der digitalen Datenübertragungseinheit (8, 9) und der Datenverarbeitungseinheit (6, 7) bidirektional austauschbar sein.

## Beschreibung

Die vorliegende Erfindung betrifft eine Anlage für die Sterilisation und Reinigung von medizinischen und pharmazeutischen Gütern sowie ein Verfahren zum Steuern einer Anlage.

Anlagen für die Aufbereitung von medizinischen und pharmazeutischen Gütern sind aus dem Stand der Technik bekannt. Im Betrieb werden Anlagen, wie beispielsweise Sterilisatoren und Reinigungsanlagen, kontinuierlich durch Sensoren überwacht. Da die Anlagen medizinische und pharmazeutische Güter sterilisieren und/oder reinigen, welche danach im Laborbereich oder direkt am Patienten eingesetzt werden, ist es von äusserster Wichtigkeit, die Qualität eines Reinigungs- oder Sterilisationsprozesses einer Anlage zu überwachen, um eine hohe und konstante Qualität des Sterilisationsprozesses oder Reinigungsprozesses zu gewährleisten. Die aus dem Stand der Technik bekannten Anlagen haben den Nachteil, dass die Sensorik mit den sich anschliessenden Messketten störungsanfällig sind und eine hohe Messunsicherheit aufweist. Eine störungsanfällige Sensorik und Messtechnik in einer Anlage ist ein grosser Nachteil, weil bei Ungenauigkeiten oder Messfehlern ein Reinigungs- und Sterilisationsprozess wiederholt werden muss, was eine Verzögerung des Prozesses bedeutet und Kosten verursacht.

Es ist die Aufgabe der Erfindung, die Nachteile des Standes der Technik zu überwinden und insbesondere eine Anlage sowie ein Verfahren zum Steuern einer Anlage zu schaffen, welches zuverlässig und in einer hohen, konstanten Qualität durchgeführt werden kann.

Die Aufgabe wird durch eine Anlage für die Sterilisation und/oder Reinigung von medizinischen und pharmazeutischen Gütern sowie durch ein Verfahren zum Steuern einer Anlage gemäss den unabhängigen Patentansprüchen gelöst.

Insbesondere wird die Aufgabe durch eine Anlage für die Sterilisation und/oder Reinigung von medizinischen und pharmazeutischen Gütern gelöst. Die Anlage umfasst eine Kammer. Die Kammer umfasst eine Medieneinbringung sowie eine Aktorregelung. Mit der Aktorregelung ist eine physikalische Grösse in der Kammer regelbar. Die physikalische Grösse kann beispielsweise Temperatur und/oder Druck und/oder ein Leitwert sein. In der Kammer ist ein Sensor angeordnet. In der Kammer kann als Sensor ein Drucksensor und/oder ein Temperatursensor und/oder ein Leitwertsensor angeordnet sein. Die Anlage weist eine digitale Datenübertragungseinheit auf. Durch die digitale Datenübertragungseinheit sind Daten des Sensors digital zu einer Datenverarbeitungseinheit übertragbar.

Durch die Medieneinbringung können Medien in die Kammer eingebracht werden. Medien können Gase und/oder Flüssigkeiten sein. Medien können beispielsweise Luft, Wasser, Dampf, Klarspüler oder Reinigungsmittel sein. Aktoren können beispielsweise Motoren oder Ventile sein. Ein Ventil kann ein Stetigventil sein. Mit der Aktorregelung können physikalische Grössen wie der Druck oder die Temperatur in der Kammer anhand von durch Sensoren gemessenen physikalischen Werten geregelt werden.

Die Anlage kann ein Sterilisator oder eine Reinigungsanlage für medizinische und pharmazeutische Güter sein. Die Kammer kann als Waschkammer ausgebildet sein. Die Daten des Sensors können drahtlos oder kabelgebunden von der digitalen Datenübertragungseinheit zu der Datenverarbeitungseinheit, insbesondere in Echtzeit, übertragbar sein. Es ist möglich, dass Daten des Sensors zwischen der digitalen Datenübertragungseinheit sowie der Datenverarbeitungseinheit bidirektional austauschbar sind.

Durch eine digitale Übertragung der Daten des Sensors durch die digitale Datenübertragungseinheit wird eine gleichbleibende und hohe Übertragungsqualität der übertragenen Daten gewährleistet. Dadurch können die Daten des Sensors vorteilhaft verarbeitet werden und die Qualität des Reinigungs- und Sterilisationsprozesses kann vorteilhaft überwacht und dokumentiert werden.

Die Anlage und insbesondere die Kammer können im Wesentlichen aus Edelstahl hergestellt sein. Es ist auch möglich, dass die Kammer aus einem anderen Material hergestellt ist. Wesentlich ist, dass die Kammer aus einem wärmebeständigen, robusten, gut zu reinigenden Material hergestellt ist. Die Kammer kann im wesentlichen quaderförmig ausgebildet sein.

Es ist möglich, dass in der Kammer mehrere Sensoren angeordnet sind. Es ist möglich, dass in der Kammer mehrere Drucksensoren und/oder mehrere Temperatursensoren und oder mehrere Leitwertsensoren angeordnet sind. Bei dem Sensor kann es sich um einen Temperatursensor, einen Drucksensor, einen Feuchtigkeitssensor oder einen Sensor handeln, mit dem ein Volumenstrom messbar ist. Auch mehrere der vorher genannten Sensoren können in der Anlage verbaut sein. Auch Sensoren, die andere physikalische Grössen messen, können in der Anlage verbaut sein. Bei der digitalen Übertragungseinheit kann es sich um einen IO-Link Slave handeln. Der IO-Link Slave kann zum Datenaustausch mit einem IO-Link Master ausgebildet sein. Die Anlage kann mit einem oder mehreren IO-Link Slaves ausgerüstet oder nachgerüstet werden. Es ist möglich, dass mehrere Sensoren zu einer Sensoreinheit zusammengefasst sind, wobei die Daten der Sensoreinheit zu mehreren IO-Link Slaves übertragen werden, wobei jeder Sensor einem IO-Link Slave zugeordnet ist. Dann ist es möglich, mehrere unabhängige Messketten mit Daten der verschiedenen Sensoren zu speisen. Dabei ist ein Sensor einer Messkette zugeordnet. Dadurch entsteht eine Redundanz der Messketten, wenn zwei Sensoren die gleiche physikalische Grösse messen und jeweils einer Messkette zugeordnet sind. Beispielsweise kann eine erste Messkette für die Steuerung oder Regelung des Drucks und/oder der Temperatur in der Kammer der Anlage verwendet werden. Es ist möglich, dass mit einer zweiten Messkette die gleiche physikalische Grösse gemessen wird wie mit der ersten Messkette. Dann können die Daten der ersten Messkette und die Daten der zweiten Messkette verglichen werden. Dies kann während des Reinigungs- und Sterilisationsprozesses geschehen. Durch einen Vergleich der Daten während des Reinigungs- und Sterilisationsprozessen kann der Prozess vorteilhaft gesteuert oder geregelt werden. Auch ein Vergleich der Daten der ersten Messkette mit den Daten der zweiten Messkette nach dem Reinigungs- und Sterilisationsprozess ist möglich.

Die weitere Messkette, welche sich an einen weiteren IO-Link Slave anschliesst, kann komplett digital ausgebildet sein. Digitale Signale sind im Gegensatz zu analogen Signalen unanfälliger für Störungen. Durch die Umwandlung von analogen Signalen in digitale Signale möglichst früh in der Messkette wird somit die Genauigkeit und Zuverlässigkeit der Messkette erhöht.

Eine drahtlose Übertragung der Daten ermöglicht es, die Daten ohne aufwendig zu verlegende Kabel zu übertragen. Durch eine drahtlose Übertragung wird die Übertragung der Daten somit vereinfacht.

Eine bidirektionale Übertragung der Daten zwischen der digitalen Datenübertragungseinheit und der Datenverarbeitungseinheit sorgt dafür, dass auch Daten von der Datenverarbeitungseinheit zu der digitalen Datenübertragungseinheit gesendet werden können. Dadurch ist es möglich, die Messkette zu kalibrieren.

Ein analoger Sensor ist ein Sensor, welcher Daten analog misst und analoge Daten zu der weiteren Messkette weiterleitet. Ein digitaler Sensor ist ein Sensor, welcher Daten analog misst und die Daten zu digitalen Daten umwandelt und die Daten digital in die weitere Messkette einspeist. Ein analoger Sensor liefert ein kontinuierliches, analoges Signal, welches sich gleichmässig mit den Änderungen der gemessenen Grösse verändert. Das Signal eines analogen Sensors kann somit jeden Wert innerhalb eines bestimmten Bereichs annehmen. Ein digitaler Sensor liefert ein diskretes, quantisiertes Ausgangssignal, das die gemessene Grösse in einem digitalen Format in Form von Binärwerten darstellt. Ein Sensor, welcher analoge Daten misst, kann mit einer Umwandlungseinheit ausgestattet werden, welche analoge Daten in digitale Daten umwandelt. Ein mit einer Umwandlungseinheit ausgestatteter Sensor, welcher digitale Daten liefert, ist ein digitaler Sensor.

Ein digitaler Sensor ist ein elektronisches Gerät, das Messwerte oder Informationen aus der physischen Umwelt erfasst und diese in digitaler Form ausgibt. Im Gegensatz zu analogen Sensoren, die kontinuierliche Signale erzeugen, erzeugen digitale Sensoren diskrete digitale Signale oder Daten, die in Form von binären Werten (0 und 1) oder digitalen Codes ausgegeben werden. Diese digitalen Daten können leicht von Mikrocontrollern, Computern und anderen digitalen Systemen verarbeitet, übertragen und analysiert werden. Digitale Sensoren bieten oft eine höhere Präzision und Genauigkeit bei der Messung von physikalischen Größen im Vergleich zu analogen Sensoren. Digitale Sensoren sind oft kleiner und kompakter, da sie weniger externe Schaltungselemente für die Signalverarbeitung benötigen. Ausserdem sind digitale Signale weniger anfällig für Störungen und Rauschen, da sie durch Fehlerkorrekturmechanismen geschützt werden können. Digitale Sensoren können leicht in digitale Systeme und Mikrocontroller integriert werden, ohne dass komplexe Analog-Digital-Wandlungen erforderlich sind. Viele digitale Sensoren sind programmierbar und ermöglichen es Benutzern, Messparameter und Konfigurationen anzupassen.

Digitale Sensoren ermöglichen eine einfache Integration von Sensordaten in digitale Netzwerke und Anwendungen.

Es ist möglich, dass es sich bei dem Sensor, beispielsweise bei dem Drucksensor und/oder bei dem Temperatursensor um einen analogen Sensor handelt und der Sterilisator eine Umwandlungseinheit aufweist, durch welche analogen Daten, die zwischen dem Sensor und der Datenübertragungseinheit übertragbar sind, in digitale Daten umwandelbar sind.

Die Umwandlungseinheit kann baulich in die Datenübertragungseinheit integriert sein. Es ist möglich, dass die Umwandlungseinheit und die Datenübertragungseinheit in ein Gehäuse integriert sind. Es ist möglich, dass die digitale Datenübertragungseinheit ein IO-Link Slave ist und die Umwandlungseinheit in den IO-Link Slave integriert ist. Dann können von einem analogen Sensor Daten analog zu dem IO-Link Slave übertragen und von der Umwandlungseinheit in dem IO-Link Slave zu digitalen Daten umgewandelt und anschliessend zu einer Datenverarbeitungseinheit übertragen werden.

Durch die Anordnung eines IO-Link Slave und die Umwandlung von analogen Signalen des Drucksensors und/oder des Temperatursensors in digitale Signale durch eine Umwandlungseinheit in dem IO-Link Slave können Anlagen, beispielsweise Sterilisatoren, welche analoge Sensoren umfassen, einfach nachgerüstet werden und bei nachgerüsteten Anlagen ist es möglich, die Daten auf einem Grossteil der Messkette digital zu übertragen. Dadurch werden Verluste in der Messkette verhindert, die Daten des Sensors, beispielsweise des Drucksensors und/oder des Temperatursensors, können vorteilhaft verarbeitet werden und eine hohe, konstante Qualität des Sterilisationsprozesses kann gewährleistet werden.

Es ist möglich, dass eine Anlage, beispielsweise ein Sterilisator, mehrere digitale Datenübertragungseinheiten aufweist. Es ist möglich, dass pro Sensor, beispielsweise pro Drucksensor und/oder Temperatursensor, eine Umwandlungseinheit und/oder eine digitale Datenübertragungseinheit ausgebildet ist.

Es ist auch möglich, dass für einen oder mehrere Sensoren mehrere Umwandlungseinheiten und/oder digitale Datenübertragungseinheiten ausgebildet sind. Die digitale Datenübertragungseinheit kann als IO-Link Slave ausgebildet sein. Wenn in der Anlage, beispielsweise in dem Sterilisator, eine digitale Datenübertragungseinheit ausgebildet ist und es sich bei dem Sensor, beispielsweise dem Drucksensor und/oder den Temperatursensor, um einen analogen Sensor handelt, werden die Daten von dem Sensor zu der Umwandlungseinheit analog übertragen. Dann werden die Daten von der Umwandlungseinheit von analogen Daten in digitale Daten umgewandelt. Die von der Umwandlungseinheit umgewandelten digitalen Daten können anschliessend von der digitalen Datenübertragungseinheit übertragen und in der weiteren Messkette verarbeitet werden. Es ist möglich, dass die digitale Datenübertragungseinheit und/oder die Umwandlungseinheit baulich mit dem Drucksensor und/oder dem Temperatursensor verbunden und/oder in ein gemeinsames Gehäuse integriert ist.

Es ist möglich, dass die Anlage, die als Sterilisator ausgebildet sein kann, einen digitalen Sensor umfasst. Durch den digitalen Sensor kann eine physikalische Grösse wie beispielsweise die Temperatur und/oder der Druck messbar sein. Der digitale Sensor kann eine digitale Datenübermittlungseinheit aufweisen. Durch die digitale Datenübermittlungseinheit können digitale Daten direkt zu einer Datenverarbeitungseinheit übermittelbar sein.

Die Ausbildung eines digitalen Sensors ermöglicht es, Daten über die gesamte Messkette, welche sich an den digitalen Sensor anschliesst, digital zu übertragen. Auf diese Weise können die Daten äusserst zuverlässig übermittelt werden und Messverluste und Ungenauigkeiten werden verhindert.

Es ist möglich, dass die Anlage mehrere digitale Sensoren umfasst. Es ist auch möglich, dass die Anlage einen oder mehrere digitale Sensoren und einen oder mehrere analoge Sensoren umfasst. Dann sind Daten der verschiedenen Sensoren vergleichbar. Auf diese Weise kann die Genauigkeit der Messung gesteigert und die Funktion der Sensoren überprüft und verifiziert werden.

Ein digitaler Sensor kann beispielsweise ein Temperatursensor, ein Feuchtigkeitssensor, ein Lichtsensor oder ein Drucksensor sein, wobei die Daten von dem digitalen Sensor jeweils digital bereitgestellt werden. Es ist auch möglich, dass die Anlage weitere digitale Sensoren umfasst, die andere physikalische Grössen erfassen. Es ist möglich, dass eine Anlage einen digitalen Sensor umfasst, wobei durch den digitalen Sensor eine physikalische Grösse, beispielsweise die Temperatur und/oder der Druck und/oder die Leitfähigkeit, gemessen wird und direkt von der digitalen Datenübermittlungseinheit des digitalen Sensors zu einer Datenverarbeitungseinheit übermittelt wird und die Anlage einen analogen Sensor umfasst, wobei die Daten des analogen Sensors analog zu einer Umwandlungseinheit gesendet und dort zu digitalen Daten umgewandelt und anschliessend zu einer digitalen Datenübertragungseinheit gesendet und von der digitalen Datenübertragungseinheit zu der Datenverarbeitungseinheit übertragen werden, zu der auch die Daten des digitalen Sensors übertragen wurden. Dann können die digitalen Daten des digitalen Sensors sowie die durch die Umwandlungseinheit digitalisierten Daten des analogen Sensors von der Datenverarbeitungseinheit verglichen werden. Durch eine derartige Ausbildung der Anlage ist es somit möglich, Daten, welche durch unterschiedliche Sensoren gemessen werden, zu vergleichen. Auf diese Weise können Abweichungen bei der Sterilisation und Reinigung in der Anlage für die Sterilisation und Reinigung rasch festgestellt werden und auch ein Defekt eines Sensors kann rasch festgestellt und behoben werden. Durch eine derartige Anordnung wird somit eine hohe, konstante Qualität des Sterilisations- und Reinigungsprozesses der Anlage gewährleistet.

Es ist möglich, dass der Sensor, beispielsweise der Drucksensor und/oder der Temperatursensor und/oder der digitale Sensor, als selbstüberwachender Sensor ausgebildet ist. Die genannten Sensoren können jeweils wie vorhergehend beschrieben die verschiedensten physikalischen Grössen erfassen.

Auf diese Weise kann die Funktion des oder der Sensoren jederzeit überprüft werden und falls Defekte auftreten, kann der jeweilige Sensor schnell repariert oder ausgetauscht werden.

Ein selbstüberwachender oder selbstkalibrierender Sensor ist ein Sensor, welcher Daten über seinen eigenen Zustand und/oder seine eigene Leistung messen und weiterleiten kann, ohne dass eine externe Überwachung notwendig ist. Es ist möglich, dass der oder die Sensoren mit einem integrierten Mikroprozessor oder Mikrocontroller ausgestattet sind. Es ist möglich, dass vom Sensor erfasste Rohdaten von dem Mikroprozessor oder den Mikrocontroller verarbeitet werden. Es ist möglich, dass von dem Mikroprozessor oder den Mikrocontroller die Rohdaten des Sensors in ein für die Übertragung der Daten geeignetes Format umgewandelt werden. Die Verarbeitung der Daten kann auch eine Filterung der Daten umfassen.

Es ist möglich, dass die Sensoren, welche als selbstüberwachende Sensoren ausgebildet sind, im Falle einer Störung ein Störungssignal an die digitale Datenübertragungseinheit, die Umwandlungseinheit oder die Datenverarbeitungseinheit senden. Auf diese Weise kann eine Störung bei der Messung in dem Sterilisator durch die Sensoren schnell erkannt werden und Defekte können schnell beseitigt werden.

Es ist möglich, dass durch den oder die selbstüberwachenden Sensoren eine Kalibrierungsprüfung durchführbar ist. Dann können der oder die selbstüberwachenden Sensoren ihre Messungen mit einem Referenzwert vergleichen. So können der oder die selbstüberwachenden Sensoren feststellen, ob die geforderte Messgenauigkeit gewährleistet ist.

Es ist möglich, dass der Sterilisator eine erste Datenverarbeitungseinheit aufweist. Es ist auch möglich, dass der Sterilisator eine zweite Datenverarbeitungseinheit aufweist. Durch die erste Datenverarbeitungseinheit und/oder die zweite Datenverarbeitungseinheit können Daten mit der Datenübertragungseinheit austauschbar sein. Es ist durch die erste Datenverarbeitungseinheit und/oder die zweite Datenverarbeitungseinheit können auch Daten mit der Datenübermittlungseinheit austauschbar sein. Der Datenaustausch zwischen der ersten Datenverarbeitungseinheit und/oder der zweiten Datenverarbeitungseinheit und der Datenübertragungseinheit und/oder der Datenübermittelungseinheit kann bidirektional erfolgen. Durch die erste Datenverarbeitungseinheit und/oder die zweite Datenverarbeitungseinheit können Daten des digitalen Sensors mit Daten des Sensors vergleichbar sein. Bei dem digitalen Sensor und bei dem Sensor kann es sich jeweils um einen Sensor des Typs handeln wir vorhergehend beschrieben. Durch die Ausbildung einer ersten Datenverarbeitungseinheit und/oder einer zweiten Datenverarbeitungseinheit können die Daten vorteilhaft verarbeitet werden. Es ist möglich, dass die erste Datenverarbeitungseinheit einer ersten Messkette zugeordnet ist und dass die zweite Datenverarbeitungseinheit einer zweiten Messkette zugeordnet ist.

Es ist möglich, dass die erste Datenverarbeitungseinheit und/oder die zweite Datenverarbeitungseinheit baulich an oder in der Anlage angeordnet sind. Es ist auch möglich, dass die erste Datenverarbeitungseinheit und/oder die zweite Datenverarbeitungseinheit baulich nicht an der Anlage, sondern in dessen Umgebung angeordnet sind. Es ist möglich, dass in der ersten Datenverarbeitungseinheit und/oder in der zweiten Datenverarbeitungseinheit auch Daten von anderen Sterilisatoren oder anderen Anlagen verarbeitet werden.

Durch einen Vergleich der Daten des Sensors mit Daten des digitalen Sensors ist es möglich, eine hohe Messgenauigkeit zu erzielen und Defekte und Abweichungen schnell zu erkennen. Wenn die Sensoren zusätzlich zu dem Vergleich der Daten durch die erste Datenverarbeitungseinheit und/oder die zweite Datenverarbeitungseinheit als selbstüberwachende Sensoren ausgebildet sind, sorgt dies für zusätzliche Redundanz und Sicherheit. Auf diese Weise entsteht ein äusserst robustes und genau arbeitendes Gesamtsystem, welches unanfällig für Fehler ist. Sollten trotzdem Fehler und Ungenauigkeiten auftreten, können diese schnell erkannt und behoben werden.

Es ist möglich, dass von einem analogen Sensor analoge Daten zu einer Umwandlungseinheit und von der Umwandlungseinheit digital zu einer digitalen Datenübertragungseinheit gesendet werden. Anschliessend können die von der Umwandlungseinheit digitalisierten Daten von der digitalen Datenübertragungseinheit an eine erste Datenverarbeitungseinheit gesendet werden. Es ist möglich, dass digitale erhobene Daten des digitalen Sensors direkt von dem digitalen Sensor an die erste Datenverarbeitungsarbeit gesendet werden. Somit enthält die erste Datenverarbeitungseinheit sowohl digitale Daten des digitalen Sensors als auch durch die Umwandlungseinheit digitalisierte Daten des analogen Sensors. Die erste Datenverarbeitungseinheit kann die Daten des digitalen Sensors sowie des analogen Sensors anschliessend vergleichen und weiterverarbeiten.

Die erste Datenverarbeitungseinheit und/oder die zweite Datenverarbeitungseinheit können als IO-Link Master ausgebildet sein. Es ist möglich, dass durch einen analogen Sensor analoge Daten zu einer weiteren Umwandlungseinheit gesendet werden. Von der weiteren Umwandlungseinheit können die Daten an eine weitere digitale Datenübertragungseinheit und von der weiteren digitalen Datenübertragungseinheit zu einer zweiten Datenverarbeitungseinheit gesendet werden. Auf diese Weise entsteht eine zweite Messkette, welche von der ersten Messkette unabhängig ist.

Ein IO-Link ist ein globaler Kommunikationsstandard für Sensoren. Die aktuelle Version ist IEC 61131-9:2022.

Es ist möglich, dass die Anlage eine Datenspeichereinheit umfasst. Durch die Datenspeichereinheit können Daten des Sensors, beispielsweise die Daten des Drucksensors und/oder Daten des Temperatursensors und/oder Daten des digitalen Sensors, speicherbar sein. Zwischen der Datenspeichereinheit und der zweiten Datenverarbeitungseinheit können Daten übertragbar sein.

Durch die Speicherung der Daten ist es möglich, eine ordnungsgemässe Sterilisation und Reinigung und eine ordnungsgemässe Funktion der Anlage zu dokumentieren.

Es ist möglich, dass die Anlage mehrere Datenspeichereinheiten umfasst. Eine Datenspeichereinheit ist speziell für die Speicherung von Daten ausgebildet. Eine Datenspeichereinheit speichert die Daten somit nicht nur temporär zur Verarbeitung, sondern ist in der Lage, die eingespeisten Daten dauerhaft zu speichern.

Es ist möglich, dass die Daten von dem zweiten Datenverarbeitungseinheit zu einer Datenspeichereinheit weitergeleitet und dort gespeichert werden. Auf diese Weise ist es möglich, die Messdaten zu speichern und die Qualität der Messung und somit die Qualität des Reinigungs- und Sterilisationsprozesses zu gewährleisten.

Es ist möglich, dass in der Anlage ein weiterer digitaler Sensor ausgebildet ist, welcher Daten digital direkt zu der zweiten Datenverarbeitungseinheit sendet. Dann empfängt die zweite Datenverarbeitungseinheit digitalisierte Daten eines analogen Sensors und digitale Daten des Weiteren digitalen Sensors. Somit können auch von der zweiten Datenverarbeitungseinheit Daten verglichen werden bzw. es können von der zweiten Datenverarbeitungseinheit sowohl digitale Daten eines digitalen Sensors als auch digitalisierte Daten eines analogen Sensors weitergeleitet und von einer Datenspeichereinheit gespeichert werden.

Es ist auch möglich, dass von einem Sensor, beispielsweise von einem Temperatursensor und/oder einem Drucksensor und/oder dem digitalen Sensor, Daten an die erste Datenverarbeitungseinheit und an die zweite Datenverarbeitungseinheit gesendet werden. Dann können Daten von sämtlichen Sensoren von der ersten Datenverarbeitungseinheit und von der zweiten Datenverarbeitungseinheit verarbeitet und weitergeleitet werden.

Die Datenübermittlung von der zweiten Datenverarbeitungseinheit zu der Datenspeichereinheit kann beispielsweise über ein Standard-Industrieprotokoll (MQTT, JSON, UPC UA) erfolgen. Die Datenspeichereinheit kann ein Computer oder ein Server sein. Die Datenübertragung von der digitalen Datenübertragungseinheit und/oder von dem digitalen Sensor zu der ersten Datenverarbeitungseinheit und/oder der zweiten Datenverarbeitungseinheit können über ein Standard-Sensorprotokoll (z.B. IO-Link) erfolgen.

Die Verwendung von solchen bekannten Übertragungstechnologien sorgt für eine zuverlässige, sichere Übertragung.

Es ist möglich, dass die Anlageeine Regelung umfasst. Zwischen der ersten Datenverarbeitungseinheit und der Regelung können Daten übertragbar sein.

Durch die Ausbildung einer Regelung ist es möglich, die Daten der Datenverarbeitungseinheiten entsprechend zu verarbeiten und den Reinigungs- oder Sterilisationsprozess entsprechend anzupassen und zu regeln.

Es ist möglich, dass durch die Regelung basierend auf den Daten des Sensors, beispielsweise des Drucksensors und/oder des Temperatursensors und/oder des digitalen Sensors, eine physikalische Grösse, beispielsweise der Druck und/oder die Temperatur und/oder die Leitfähigkeit in der Kammer, steuerbar ist. Es ist auch möglich, dass durch die Regelung basierend auf den Daten des Sensors eine physikalische Grösse, beispielsweise der Druck und/oder die Temperatur, in der Kammer regelbar ist.

Die Kammer der Anlage kann eine Reinigungs- und/oder Sterilisationskammer sein.

Durch eine Regelung einer physikalischen Grösse, beispielsweise durch eine Regelung des Drucks und/oder der Temperatur, in der Kammer oder durch eine Steuerung einer physikalischen Grösse, beispielsweise durch eine Steuerung des Drucks und/oder der Temperatur in der Kammer ist es möglich, den Reinigungs- und Sterilisationsprozess zu steuern bzw. zu regeln. Dadurch wird im Wesentlichen unabhängig von äusseren Rahmenbedingungen eine konstante hohe Qualität des Reinigungs- und Sterilisationsprozesses gewährleistet.

Die Aufgabe der Erfindung wird ausserdem durch ein Verfahren zum Steuern, insbesondere Regeln, einer Anlage wie vorhergehend beschrieben gelöst. Das Verfahren umfasst die folgenden Schritte:
- Messen einer physikalischen Grösse, insbesondere Messen von Druck und/oder Temperatur und/oder Leitfähigkeit in der Kammer durch den Sensor,
- Übermittlung der Daten von dem Sensor zu der Datenübertragungseinheit,
- Digitale Übertragung der Daten des Sensors von der Datenübertragungseinheit zu der ersten Datenverarbeitungseinheit.

Ein solches Verfahren ist unkompliziert und störungsfrei durchführbar und hat im Wesentlichen die gleichen Vorteile wie eine Anlage wie vorhergehend beschrieben.

Das Verfahren kann die folgenden weiteren Schritte umfassen:
- Übermittlung der digitalen Daten durch die erste Datenverarbeitungseinheit an eine Regelung,
- Insbesondere Regelung einer physikalischen Grösse und insbesondere der Temperatur und/oder des Drucks in der Anlage durch die Regelung,
- Insbesondere manipulationssichere Speicherung der Daten durch eine Datenspeichereinheit.

Durch eine derartige engmaschige Regelung des Reinigungs- und Sterilisationsprozesses kann eine hohe, gleichbleibende Qualität des Reinigungs- und Sterilisationsprozesses gewährleistet werden. Ein solches Verfahren hat somit im Wesentlichen dieselben Vorteile wie eine Anlagewie vorhergehend beschrieben.

Das Verfahren kann die folgenden weiteren Schritte umfassen:
- Übermittlung von digitalen Daten des digitalen Sensors an die erste Datenverarbeitungseinheit durch die Datenübermittlungseinheit,
- Übermittlung der digitalen Daten des digitalen Sensors von der ersten Datenverarbeitungseinheit an die Regelung.

Durch diese Verfahrensschritte ist es möglich, Daten des digitalen Sensors und der weiteren Sensoren zu vergleichen und die Anlage entsprechend zu regeln. Die Regelung der Anlage ist somit sehr unanfällig für Störungen. Die Verfahrensschritte haben im Wesentlichen dieselben Vorteile wie eine Anlagewie vorhergehend beschrieben.

Das Verfahren kann die folgenden weiteren Schritte umfassen:
- Vergleich der digitalen Daten des digitalen Sensors mit den Daten des Sensors durch die erste Datenverarbeitungseinheit,
- Falls bei dem Vergleich eine Abweichung festgestellt werden sollte: Senden eines Abweichungssignals an die Regelung,
- Insbesondere Senden eines Anzeigesignals durch die Regelung an eine Anzeigevorrichtung, wenn die Regelung ein Abweichungssignal von der ersten Datenverarbeitungseinheit empfängt,
- Insbesondere Senden eines Regelungssignals von der Regelung zu der Aktorregelung, wenn die Regelung ein Abweichungssignal empfängt.

Durch das Anzeigen einer Abweichung durch eine Anzeigevorrichtung ist es möglich, dass eine Abweichung von einem Benutzer rasch erkannt wird. Durch ein derartiges Erkennen der Abweichung durch einen Benutzer, kann der Benutzer schnell weitere Massnahmen ergreifen und beispielsweise die Anlage stoppen und einen Sensor austauschen.

Es ist möglich, dass die erste Datenverarbeitungseinheit die Daten des Sensors, beispielsweise des Temperatursensors und/oder des Drucksensors und/oder des digitalen Sensors, mit Daten eines Sterilisationsprogramms vergleicht. Es ist möglich, dass die erste Datenverarbeitungseinheit bei einer Abweichung zwischen den Daten des Sensors, beispielsweise des Temperatursensors und/oder des Drucksensors und/oder des digitalen Sensors und den Daten des Sterilisationsprogramms ein Abweichungssignal an die Regelung und/oder ein Anzeigesignal an die Anzeigevorrichtung sendet. Es ist möglich, dass die Regelung basierend auf dem Abweichungssignal, welches auf der Differenz zwischen den Sensordaten und den Daten des Sterilisationsprogramms beruht, ein Regelungssignal an die Aktorregelung sendet.

Das Verfahren kann die folgenden weiteren Schritte umfassen:
- Übertragung der Daten des Sensors von der Datenübertragungseinheit zu der zweiten Datenverarbeitungseinheit,
- Senden der Daten von der zweiten Datenverarbeitungseinheit zu der Datenspeichereinheit,
- Speichern der Daten durch die Datenspeichereinheit.

Durch derartige Verfahrensschritte können die Daten gespeichert und der Reinigungs- oder Sterilisationsprozess somit dokumentiert werden. Ein derartiges Verfahren hat somit dieselben Vorteile wie eine Anlage wie vorhergehend beschrieben.

Es ist möglich, dass ein oder mehrere der vorhergehend genannten Verfahrensschritte wiederholt durchgeführt werden.

Die Erfindung wird in den folgenden Figuren anhand von Ausführungsbeispielen näher erläutert. Es zeigen:
- Figur 1:: Ein Sterilisator mit einer Regelung und einem Anbaumodul,
- Figur 2:: einen Sterilisator mit einem Bus-Knoten,
- Figur 3:: einen Sterilisator mit einer ersten digitalen Datenübertragungseinheit und einer zweiten digitalen Datenübertragungseinheit,
- Figur 4:: einen Sterilisator mit einer ersten digitalen Datenübertragungseinheit und einer zweiten digitalen Datenübertragungseinheit sowie einem digitalen Sensor.

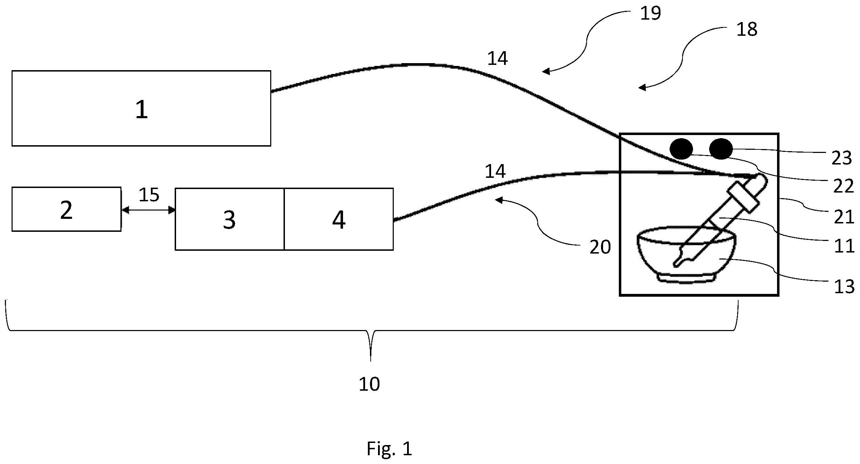

In den Figuren 1 - 4 ist für die Erläuterung der Erfindung jeweils ein Sterilisator mit den entsprechenden weiteren Komponenten dargestellt. Die Erfindung ist jedoch nicht auf einen Sterilisator beschränkt und kann beispielsweise auch eine Anlage für die Reinigung von medizinischen und pharmazeutischen Gütern umfassen.

Die Figur 1 zeigt einen Sterilisator 18 mit einer Regelung 3 und einem Anbaumodul 4. Der erste Sensor 11 misst Daten an der Messstelle 13. Die Messstelle ist in der Kammer 21 angeordnet. Die Kammer 21 weist eine Medieneinbringung 22 sowie eine Aktorregelung 23 auf. Der erste Sensor 11 ist als analoger Sensor 11 ausgebildet. Der erste Sensor 11 ist mittels zweier analoger Datenübertragungswege 14 mit der Datenspeichereinheit 1 sowie mit dem Anbaumodul 4 verbunden. Das Anbaumodul 4 ist mit der Regelung 3 verbunden und baulich an die Regelung 3 angeschlossen. Durch das Anbaumodul 4 werden analogen Daten des analogen Sensors 11, welche von dem analogen Sensor 11 mittels analoger Übertragung 14 zu dem Anbaumodul übertragen werden, digitalisiert und an die Regelung 3 gesendet. Von der Regelung 3 werden die digitalen Daten mittels digitaler Übertragung 15 zu der Anzeigevorrichtung 2 gesendet. Es ist eine zweite Messkette 19 ausgebildet, bei der Daten an der Messstelle 13 in der Kammer 21 von dem analogen Sensor 11 gemessen und durch eine analoge Übertragung 14 zu der Datenspeichereinheit 1 gesendet werden. Die zweite Messkette 19 dient dazu, die Daten, welche durch den analogen Sensor 11 erfasst werden, durch die Datenspeichereinheit 1 zu speichern und den Sterilisationsprozess zu dokumentieren. Neben der zweiten Messkette 19 ist die von der zweiten Messkette 19 unabhängige erste Messkette 20 ausgebildet. Bei der ersten Messkette 20 werden Daten von dem analogen Sensor 11 an der Messstelle 13 in der Kammer 21 gemessen und durch die analoge Übertragung 14 zu dem Anbaumodul 4 übertragen. In dem Anbaumodul 4 werden die Daten in der ersten Messkette 20 digitalisiert und an die Regelung 3 gesendet. Von der Regelung 3 werden die Daten in der ersten Messkette 20 digital mittels digitale Übertragung 15 zu der Anzeigevorrichtung 2 gesendet. Es ist möglich, dass die Anzeigevorrichtung 2 als Bedienpanel ausgebildet ist. Dann kann die Anzeigevorrichtung 2 sowohl Daten anzeigen als auch Benutzerbefehle eines Benutzers annehmen und verarbeiten. In der ersten Messkette 20 werden die Daten somit für die Steuerung oder Regelung des Sterilisators 18 sowie für die Darstellung der Daten durch die Anzeigevorrichtung 2 verwendet. Sowohl die erste Messkette 20 als auch die zweite Messkette 19 liegen auf der Kalibrierstrecke 10. Somit können sowohl die erste Messkette 20 als auch die zweite Messkette 19 kalibriert werden.

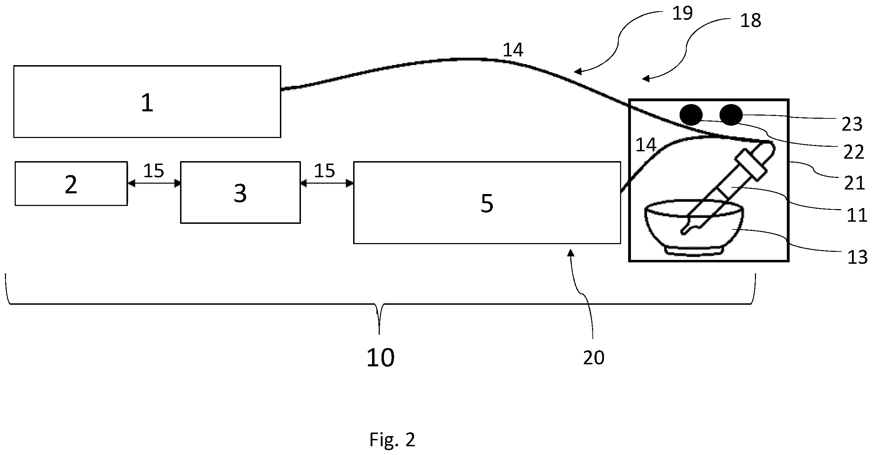

Die Figur 2 zeigt einen Sterilisator 18 mit einem Bus-Knoten 5. Der Sterilisator 18 ist analog zu dem Sterilisator 18 aus der Figur 1 ausgebildet. Anders als der Sterilisator 18 in der Figur 1 umfasst der Sterilisator 18 in der Figur 2 einen Bus-Knoten 5. Der Bus-Knoten 5 befindet sich in der ersten Messkette 20. Durch den Busknoten 5 werden die Daten, welche mittels analoger Übertragung 14 von dem analogen Sensor 11 zu dem Busknoten 5 gesendet werden, digitalisiert. Die von den Busknoten 5 digitalisierten Daten werden mittels digitaler Übertragung 15 zu der Regelung 3 gesendet. Die weiteren Vorrichtungen und Datenübertragungswege sind analog zu dem Sterilisator 18 in der Figur 1 ausgebildet.

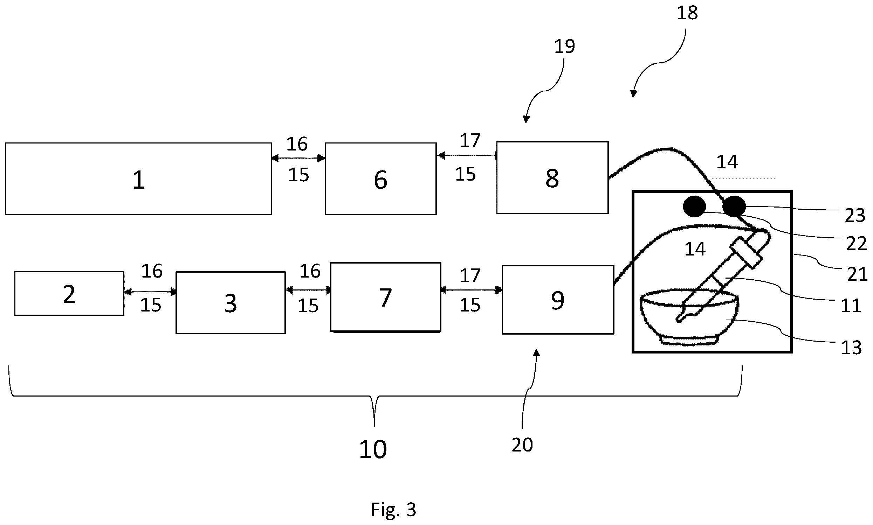

Die Figur 3 zeigt einen Sterilisator 18 mit einer ersten digitalen Datenübertragungseinheit 9 und einer zweiten digitalen Datenübertragungseinheit 8. Durch den analogen Sensor 11 werden Daten an der Messstelle 13 in der Kammer 21 erhoben und mittels analoger Übertragung 14 zu der ersten digitalen Datenübertragungseinheit 9 und zu der zweiten digitalen Datenübertragungseinheit 8 gesendet. Sowohl die erste digitale Datenübertragungseinheit 9 als auch die zweite digitale Datenübertragungseinheit 8 sind als IO-Link Slave ausgebildet. Sowohl in der ersten digitalen Datenübertragungseinheit 9 als auch in der zweiten Datenübertragungseinheit 8 ist jeweils eine Umwandlungseinheit (nicht dargestellt) integriert, durch die analoge Daten in digitale Daten umwandelbar sind. Sowohl die erste Datenverarbeitungseinheit 7 als auch die zweite digitale Datenverarbeitungseinheit 6 sind als IO-Link Master ausgebildet. Von der zweiten digitalen Datenübertragungseinheit 8 werden die Daten mittels digitaler Übertragung 15 mittels eines Sensorprotokolls 17 an die zweite digitalen Datenverarbeitungseinheit 6 gesendet. Durch die zweite digitale Datenverarbeitungseinheit 6 werden die Daten mittels eines Standard-Industrie Protokolls 16 mittels digitaler Übertragung 15 an die Datenspeichereinheit 1 gesendet.

Von der ersten digitalen Datenübertragungseinheit 9 werden die Daten mittels digitaler Übertragung 15 mittels eines Sensorprotokolls 17 an eine erste digitale Datenverarbeitungseinheit 7 gesendet. Von der ersten digitalen Datenverarbeitungseinheit 7 werden die Daten mittels digitaler Übertragung 15 mittels eines Standard Industrie Protokolls 16 zu der Regelung 3 gesendet. Von der Regelung 3 werden die Daten mittels digitaler Übertragung 15 mittels eines Standard-Industrie Protokolls 16 an die Anzeigevorrichtung 2 gesendet. Der Datenfluss von dem analogen Sensor 11 zu der ersten digitalen Datenübertragungseinheit 9, der ersten Datenverarbeitungseinheit 7, der Regelung 3 sowie der Anzeigevorrichtung 2 bilden die erste Messkette 20. Die erste Messkette 20 dient somit der Regelung des Sterilisationsprozesses in dem Sterilisator 18 sowie der Anzeige von Daten durch die Anzeigevorrichtung 2, welche zusätzlich als Bedienpanel ausgebildet sein kann.

Der Datenfluss von dem analogen Sensor 11 zu der zweiten digitalen Datenübertragungseinheit 8, der zweiten Datenverarbeitungseinheit 6 sowie der Datenspeichereinheit 1 bildet die zweite Messkette 19. Die zweite Messkette 19 dient der Speicherung der Daten des analogen Sensors 11. Die erste Messkette 20 und die zweite Messkette 19 sind unabhängig voneinander ausgebildet. Sowohl die erste Messkette 20 als auch die zweite Messkette 19 liegen auf der Kalibrierstrecke 10. Somit ist sowohl die erste Messkette 20 als auch die zweite Messkette 19 kalibrierbar.

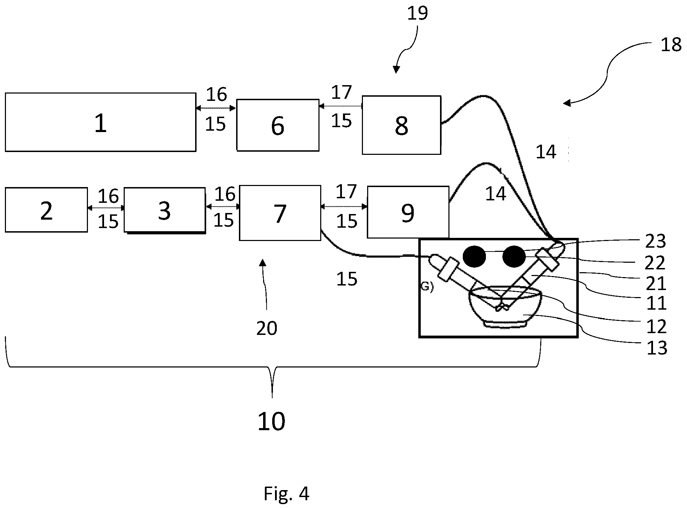

Die Figur 4 zeigt einen Sterilisator 18 mit einer ersten digitalen Datenübertragungseinheit 9, einer zweiten digitalen Datenübertragungseinheit 8 sowie einem digitalen Sensor 12. Der Sterilisator 18 in der Figur 4 ist analog ausgebildet zu dem Sterilisator 18 aus der Figur 3. Anders als der Sterilisator 18 aus der Figur 3 weist der Sterilisator 18 in der Figur 4 einen digitalen Sensor 12 auf. Der digitale Sensor 12 misst wie der analoge Sensor 11 Daten an der Messstelle 13. Durch den analogen Sensor 11 und durch den digitalen Sensor 12 werden somit die gleichen Daten erhoben. Die Daten, welche der digitale Sensor 12 misst, werden mittels digitaler Übertragung 15 an die erste Datenverarbeitungseinheit 7 gesendet. Die erste Datenverarbeitungseinheit 7 erhält somit digitale Daten des digitalen Sensors 12 mittels digitaler Übertragung 15. Ausserdem erhält die erste Datenverarbeitungseinheit 7 Daten des analogen Sensors 11, welche durch die erste digitale Datenübertragungseinheit digitalisiert werden. Somit erhält die erste Datenverarbeitungseinheit 7 in der ersten Messkette 20 Daten von sowohl dem analogen Sensor 11 als auch von dem digitalen Sensor 12. Die Daten des analogen Sensors 11 sowie die Daten des digitalen Sensors 12 können somit von der ersten Datenverarbeitungseinheit 7 verglichen und von der Regelung 3 verarbeitet werden. Die Daten an der Messstelle 13 werden somit durch verschiedene Sensoren 11, 12 gemessen und in der ersten Messkette 20 verarbeitet.

## Patentansprüche

1. Anlage (18) für die Sterilisation und/oder Reinigung von medizinischen und pharmazeutischen Gütern, wobei die Anlage (18) eine Kammer (21) umfasst, wobei die Kammer(21) eine Medieneinbringung (22) sowie eine Aktorregelung (23) umfasst, wobei mit der Aktorregelung (23) eine physikalische Grösse, insbesondere der Druck und/oder die Temperatur, in der Kammer (21) regelbar ist, wobei in der Kammer (21) ein Sensor, insbesondere ein Drucksensor (11, 12) und/oder ein Temperatursensor (11, 12), angeordnet ist, **dadurch gekennzeichnet, dass** die Anlage (18) eine digitale Datenübertragungseinheit (8, 9) aufweist, durch welche Daten des Sensors (11, 12), insbesondere kabelgebunden oder drahtlos, bevorzugt bidirektional, digital (15) zu einer Datenverarbeitungseinheit (6,7) übertragbar sind.

2. Anlage (18) nach Anspruch 1, wobei es sich bei dem Sensor (11, 12) um einen analogen Sensor (11) handelt und die Anlage (18) eine Umwandlungseinheit (8, 9) aufweist, durch welche analoge Daten, die zwischen dem Sensor (11, 12) und der Datenübertragungseinheit (8, 9) übertragbar sind, in digitale Daten umwandelbar sind.

3. Anlage (18) nach einem der vorhergehenden Ansprüche, wobei die Anlage (18) einen digitalen Sensor (12) umfasst, wobei durch den digitalen Sensor (12) eine physikalische Grösse und insbesondere die Temperatur und/oder der Druck messbar ist, wobei der digitale Sensor (11) eine digitale Datenübermittlungseinheit aufweist, durch welche digitale Daten direkt zu einer Datenverarbeitungseinheit (6, 7) übermittelbar sind.

4. Anlage (18) nach einem der vorhergehenden Ansprüche, wobei der Sensor und/oder der digitale Sensor (12) als selbstüberwachender Sensor ausgebildet ist.

5. Anlage (18) nach einem der vorhergehenden Ansprüche, wobei die Anlage (18) eine erste Datenverarbeitungseinheit (7) und insbesondere eine zweite Datenverarbeitungseinheit (6) aufweist, durch welche Daten mit der Datenübertragungseinheit (8, 9) und insbesondere mit der Datenübermittlungseinheit, bevorzugt bidirektional, austauschbar sind, wobei durch die erste Datenverarbeitungseinheit (7) insbesondere Daten des digitalen Sensors (12) mit Daten des Sensors (11, 12) vergleichbar sind.

6. Anlage (18) nach Anspruch 5, wobei die Anlage (18) eine Datenspeichereinheit (1) umfasst, durch welche Daten des Sensors (11, 12) und/oder Daten des digitalen Sensors (12) speicherbar sind, wobei zwischen der Datenspeichereinheit (1) und der zweiten Datenverarbeitungseinheit (6) Daten übertragbar sind.

7. Anlage (18) nach einem der Ansprüche 5 bis 6, wobei die Anlage (18) eine Regelung (3) umfasst, wobei Daten zwischen der ersten Datenverarbeitungseinheit (7) und der Regelung (3) übertragbar sind.

8. Anlage (18) nach Anspruch 7, **dadurch gekennzeichnet, dass** durch die Regelung (3) basierend auf den Daten des Sensors (11, 12) und/oder des digitalen Sensors (12) eine physikalische Grösse, insbesondere der Druck und/oder die Temperatur in der Kammer (21) steuerbar, insbesondere regelbar, ist.

9. Verfahren zum Steuern, insbesondere Regeln, einer Anlage (18) nach einem der Ansprüche 1 - 8 umfassend die folgenden Schritte:
- Messen einer physikalischen Grösse, insbesondere Messen von Druck und/oder Temperatur in der Kammer (21) durch den Sensor (11, 12,
- Übermittlung der Daten von dem Sensor (11, 12) zu der Datenübertragungseinheit (8, 9),
- Digitale Übertragung (15) der Daten des Sensors (11, 12) von der Datenübertragungseinheit (8, 9) zu der ersten Datenverarbeitungseinheit (7).

10. Verfahren nach Anspruch 9, umfassend die weiteren Schritte:
- Übermittlung (15) der digitalen Daten durch die erste Datenverarbeitungseinheit (7) an eine Regelung (3),
- Insbesondere Regelung einer physikalischen Grösse und insbesondere der Temperatur und/oder des Drucks in der Anlage (18) durch die Regelung (3),
- Insbesondere, manipulationssichere Speicherung der Daten durch eine Datenspeichereinheit (1).

11. Verfahren nach einem der Ansprüche 9 bis 10, umfassend die weiteren Schritte:
- Übermittlung (15) von digitalen Daten des digitalen Sensors (12) an die erste Datenverarbeitungseinheit (7) durch die Datenübermittlungseinheit,
- Übermittlung (15) der digitalen Daten des digitalen Sensors (12) von der ersten Datenverarbeitungseinheit (7) an die Regelung (3).

12. Verfahren nach Anspruch 11, umfassend die weiteren Schritte:
- Vergleich der digitalen Daten des digitalen Sensors (12) mit den Daten des Sensors (11, 12) durch die erste Datenverarbeitungseinheit (7),
- Falls bei dem Vergleich eine Abweichung festgestellt werden sollte: Senden eines Abweichungssignals an die Regelung (3),
- Insbesondere Senden eines Anzeigesignals durch die Regelung (3) an eine Anzeigevorrichtung (2), wenn die Regelung (3) ein Abweichungssignal von der ersten Datenverarbeitungseinheit (7) empfängt,
- Insbesondere senden eines Regelungssignals von der Regelung (3) zu der Aktorregelung (23), wenn die Regelung (3) ein Abweichungssignals empfängt.

13. Verfahren nach einem der Ansprüche 9 bis 12, umfassend die folgenden Schritte:
- Übertragung der Daten des Sensors (11, 12) von der Datenübertragungseinheit (8, 9) zu der zweiten Datenverarbeitungseinheit (6),
- Senden der Daten von der zweiten Datenverarbeitungseinheit (6) zu der Datenspeichereinheit (1),
- Speichern der Daten durch die Datenspeichereinheit (1).
